# EUROPEAN PATENT SPECIFICATION

(11) **EP 4 032 562 B1**
(45) Date of publication and mention of the grant of the patent: **18.12.2024**
(21) Application number: 20865239.6
(22) Date of filing: 31.03.2020
(51) Int. Cl.: A61L 31/04, A61L 31/14, C08B 37/00, A61P 41/00, C08J 3/24, C08B 37/08

(54) **DISULFIDE BOND-LINKED HYALURONIC ACID GEL FOR PREVENTING TISSUE ADHESION AFTER ABDOMINAL OR PELVIC CAVITY SURGERY AND PREPARATION METHOD THEREFOR**
DISULFIDGEBUNDENES HYALURONSÄUREGEL ZUM VERHINDERN VON GEWEBEANHAFTUNGEN NACH CHIRURGISCHEN EINGRIFFEN IN DER UNTERLEIBS- ODER BECKENHÖHLE UND HERSTELLUNGSVERFAHREN DAFÜR
GEL D'ACIDE HYALURONIQUE À LIAISON DISULFURE POUR PRÉVENIR L'ADHÉRENCE TISSULAIRE APRÈS UNE CHIRURGIE DE LA CAVITÉ ABDOMINALE OU PELVIENNE, ET SON PROCÉDÉ DE PRÉPARATION

(30) Priority: 21.09.2019 CN 201910898102
(43) Date of publication of application: 27.07.2022
(73) Proprietor: Bioregen Biomedical (Changzhou) Co., Ltd., Changzhou, Jiangsu 213125 (CN)
(72) Inventor: WANG, Yunyun, Changzhou, Jiangsu 213125 (CN); ZHANG, Hongchen, Changzhou, Jiangsu 213125 (CN); WANG, Kun, Changzhou, Jiangsu 213125 (CN); HU, Mulan, Changzhou, Jiangsu 213125 (CN); WANG, Xinyu, Changzhou, Jiangsu 213125 (CN); SONG, Wenjun, Changzhou, Jiangsu 213125 (CN); SHU, Xiaozheng, Changzhou, Jiangsu 213125 (CN)
(74) Representative: Grünecker Patent- und Rechtsanwälte PartG mbB
(86) International application number: PCT/CN2020/082597
(87) International publication number: WO 2021/051778

(56) References cited:
- CN-A- 101 137 388
- CN-A- 101 338 036
- CN-A- 101 721 349
- CN-A- 102 399 295
- CN-A- 110 787 325
- US-A1- 2013 338 099
- US-A1- 2017 232 148

## Description

### Technical field

The present invention relates to the field of biomedicine, in particular to a disulfide crosslinked hyaluronic acid gel for postoperative abdominal (pelvic) adhesion prevention, and also relates to the method for preparing disulfide crosslinked hyaluronic acid gel for postoperative abdominal (pelvic) adhesion prevention.

### Background

Postoperative adhesion is an unavoidable pathophysiological phenomenon that occurs during natural healing. There are fibrous bands formed by scar tissue in vivo, resulting in the abnormal connection between normal tissues or organs which should not be sticked together. Tissue adhesion is usually the most severe after abdominal (pelvic) operations, and the incidence can be as high as 90%. Postoperative complications include bowel obstruction, secondary infertility, and abdominal (pelvic) pain. (Sikirica et al., BMC Surg. 2011, 11:13; ten Broek et al., BMJ 2013, 347: f5588).

Although every effort should be made during operations to prevent the formation of postoperative adhesions, adhesions are part of the natural healing process, and even meticulous surgical techniques are not sufficient to prevent adhesions. In addition, to date, there has not been any marketed treatment or "specific medicine" that can reduce adhesions after they have formed. Many patients with adhesion-related complications need another operation to diagnosis. If adhesions from the previous operation are found, adhesiolysis has to be performed to lysis adhesions. Although adhesiolysis can be a solution for some patients, the adhesion reformed frequently. Therefore, preventive adhesion prevention strategies and the development of safe and effective adhesion prevention products have are the current focus (DeWilde et al., Gynecol Surg. 2007, 4: 243-253).

Adhesion Barrier can physically isolate the injured tissue during its repair process, so theoretically it can effectively reduce the formation of adhesions. The ideal adhesion barrier should be safe, degradable and absorbable in vivo, suitable for application after both laparotomy and laparoscopic surgical methods, and can effectively prevent the formation of postoperative abdominal (pelvic) adhesions. At present, several adhesion barriers have been approved for use after abdominal (pelvic) operation, but they differ in application, efficacy and safety, and significant improvement is still needed (Tulandi et al., Curr Opin Obstet Gynecol .2005, 17: 395-398; DeWilde et al., Gynecol Surg. 2007, 4: 243-253; Arung et al., World J Gastroenterol. 2011, 17: 4545-4553; Practice Committee of the American Society for Reproductive Medicine in collaboration with the Society of Reproductive Surgeons, Fertil Steril. 2013, 99: 1550-1555).

Hyaluronic acid is a non-sulfonated glycosaminoglycan composed of repeating disaccharide units (α-1,4-D-glucuronic acid and β-1,3-N-acetyl-D-glucosamine), which is the main component of the extracellular matrix in connective tissues and has good biocompatibility, unique physical and chemical properties and biological functions that can promote wound healing. Hyaluronic acid once had been considered as an ideal adhesion barrier. However due to its fluidity and rapid degradation by hyaluronidase in vivo, hyaluronic acid cannot physically isolate the injured tissue effectively during its repair process, and therefore cannot significantly prevent adhesion (Wiseman, In: diZeraga GS., Editor. Peritoneal Surgery. New York: Springer-Verlag; 2000, pp 401-417).

Cross-linking reaction can significantly reduce the fluidity of hyaluronic acid and delay its degradation and absorption in vivo. Cross-linked hyaluronic acid may effectively isolate the injured tissue during its repair process. Thus it has good potential in preventing adhesions after abdominal (pelvic) operation. However, improper cross-linking reactions may also impair the biocompatibility of hyaluronic acid. For example, trivalent ferric ion cross-linked sodium hyaluronate gel has caused many serious adverse events (Wiseman, Ann Surg. 2006, 244: 630 -632).

In a prior patent document (CN 102399295A) the applicant of the present invention discloses a disulfide crosslinked hyaluronic acid gel, wherein the original structure, physiological function and biocompatibility of hyaluronic acid is maintained to the maximum extent, and at the same time the degradation, absorption and dissolution fluidity of hyaluronic acid in vivo is retarded, but its application to prevent adhesions after abdominal (pelvic) operation has not been studied in detail.

### Summary of the invention

The invention provides a disulfide crosslinked hyaluronic acid gel for preventing tissue adhesion after abdominal (pelvic) operation, which has good biocompatibility and significant effect in prevent adhesion after abdominal (pelvic) operation.

The scope of the invention is defined by the claims. Any references in the description to methods of treatment refer to the products of the present invention for use in a method for treatment.

The invention is realized through the following technical solutions:
A disulfide crosslinked hyaluronic acid gel for preventing tissue adhesion after abdominal (pelvic) operation, which is characterized in that the content of the disulfide crosslinked hyaluronic acid in the gel is between 3 ~ 4.5 mg /mL.

Adhesion barrier prevents adhesion by physically isolating the injured tissue during its repair process. As known to those skilled in the art, the strength of the cross-linked hyaluronic acid gel increases with the content of the cross-linked hyaluronic acid, which can provide a better physical isolation effect, and therefore should have a better adhesion prevention effect. For those skilled in the art, in order to further improve the adhesion prevention effect of the disulfide crosslinked hyaluronic acid gel, the technical solution that can be foreseen by those skilled in the art is to increase the content of the disulfide crosslinked hyaluronic acid.

A prior patent document (CN 102399295A) discloses that disulfide-linked cross-linked hyaluronic acid gel (10 mg / mL) has a better abdominal (pelvic) adhesion prevention effect than a non-cross-linked hyaluronic acid solution. Therefore, it can be expected according to common knowledge that a higher content of disulfide-linked crosslinked hyaluronic acid gel can further enhance the effect of abdominal (pelvic) adhesion prevention (Yang et al., BMC Biotechnology 2010, 10:65; De laco et al., Fertil Steril. 1998, 69: 318-323).

However, contrary to common knowledge that well known to those skilled in the art, the inventors of the present application found that the disulfide-linked crosslinked hyaluronic acid gel with lower content of disulfide-linked crosslinked hyaluronic acid (3 - 8 mg / mL) has better effect in preventing postoperative abdominal (pelvic) adhesion than that with higher content (10mg/mL), while it also has good biocompatibility.

The technical solution adopted by the present invention is beyond common sense, and the beneficial effects obtained are unexpected.

Another object of the present invention is to provide a method for preparing the disulfide cross-linked hyaluronic acid gel for preventing tissue adhesion after abdominal (pelvic) operation. The technical scheme of the preparation method is to obtain the disulfide crosslinked hyaluronic acid gel of the present invention through an oxidation process of an aqueous solution of a hyaluronic acid thiolated derivative.

The above-mentioned oxidation process can generally be performed under the action of oxygen, such as oxygen in the air and / or oxygen dissolved in an aqueous solution. In this process, without adding a crosslinking agent, the thiol group is oxidized to form disulfide bond, with a by-product of water and no further purification required. In the above-mentioned preparation process, the aqueous solution of the hyaluronic acid thiolated derivative can be sterilized through filtration, and then the disulfide crosslinked hyaluronic acid gel of the present invention can be prepared under aseptic conditions. Alternatively, or additionally, terminal sterilization method etc. also can be used for sterilization. The terminal sterilization method includes moist heat sterilization, which are well known to those skilled in the art.

In the present invention, the hyaluronic acid thiolated derivative refers to hyaluronic acid derivatives containing a thiol group, which can be prepared by thiolation modification of hyaluronic acid, and also includes thiolated derivatives prepared by various hyaluronic acid derivatives (such as carboxymethylation hyaluronic acid, acetylated hyaluronic acid, etc.) through further thiolation modification. The side chain carboxyl group, side chain hydroxyl group, and reducing end group of hyaluronic acid or its derivative thereof are usually active functional groups capable of thiolation modification, for example, various preparation methods of the hyaluronic acid thiolated derivatives disclosed in prior art documents such as patent document WO 2009 / 006780A1 can be used to prepare the hyaluronic acid thiolated derivatives in the present invention. Those hyaluronic acid thiolated derivatives preferably maintains the original structure, physiological function, and biocompatibility of the hyaluronic acid, and can achieve effective disulfide cross-linking, significantly retard the degradation, absorption and fluidity in vivo.

In the present invention, hyaluronic acid thiolated derivatives with various thiol contents can be used to prepare the disulfide crosslinked hyaluronic acid of the present invention. The thiol content is expressed as the number of micromoles of thiol per gram of hyaluronic acid thiolated derivative (µmol / g). The thiol content is preferably 10 to 100 µmol / g, and is particularly preferably 20 to 70 µmol / g.

In the present invention, the hyaluronic acid also includes its salt form (such as sodium salt, potassium salt, zinc salt, calcium salt, etc.).

The beneficial effects of the present invention are:
The disulfide crosslinked hyaluronic acid gel for preventing tissue adhesion after abdominal (pelvic) operation according to the present invention adopts a technical solution contrary to the common knowledge well known to those skilled in the art, which not only has a good biocompatibility but also has achieved unexpected prevention effect of tissue adhesion after abdominal (pelvic) operation. The preparation method according to the present invention has many advantages, such as no need to add a cross-linking agent, has a simple preparation process, and has no impurities etc.

### BRIEF DESCRIPTION OF THE DRAWINGS

Figure 1. Effect of disulfide crosslinked hyaluronic acid content on gel dynamic viscosity.
Figure 2. Effect of disulfide crosslinked hyaluronic acid content on the area involved in adhesion.
Figure 3. Effect of disulfide crosslinked hyaluronic acid content on the severity of adhesions.
Figure 4. Effect of the content of disulfide crosslinked hyaluronic acid on the adhesion length.

### DETAILED DESCRIPTION

The embodiments of the present invention will be described in detail below with reference to examples.

In the following examples, only compositions comprising a disulfide crosslinked hyaluronic acid having a content of disulfide crosslinked hyaluronic acid between 3-4.5 mg/mL are according to the claims, compositions comprising different concentrations are reference or comparative examples.

### Example 1: Preparation of Disulfide Crosslinked Hyaluronic Acid Gels

Hyaluronic acid thiolated derivatives are made from sodium hyaluronate with a molecular weight of 180 KDa and prepared by the method reported by Shu et al. (Shu et al., Biomacromolecules 2002, 3: 1304-1311). The thiol contents of the derivatives were 24 µmol / g, 38 µmol / g and 57 µmol / g, respectively.

The above-mentioned hyaluronic acid thiolated derivatives were dissolved to obtain aqueous solutions with contents of 3 mg / mL, 4 mg / mL, 4.5 mg / mL, 5 mg / mL, 6 mg / mL, 7 mg / mL, 8 mg / mL, and 10 mg / mL respectively. The pH value of the solutions was adjusted to 7.4. After sterilization by filtration, the solutions were transferred to sterile glass containers. The solutions were kept sealed at room temperature for 4 weeks, and the solutions lose fluidity and form disulfide crosslinked hyaluronic acid gels.

### Example 2: Evaluation of Dynamic Viscosity Properties of Disulfide Crosslinked Hyaluronic Acid Gels

The dynamic viscosity properties of the disulfide crosslinked hyaluronic acid gels prepared in Example 1 were evaluated with a rotational viscometer at a shear rate of no less than 0.25 Hz (25 ± 0.1 ° C) in accordance with the second method of Appendix VIG of Part II of the Chinese Pharmacopoeia (2010 edition).

Visual inspection showed that gels with higher contents of disulfide crosslinked hyaluronic acid had better strength, while test results also showed that gels with higher content had higher dynamic viscosity. The effect of the contents of disulfide-crosslinked hyaluronic acid on the dynamic viscosity of the gel was shown in Figure 1. The gel with disulfide crosslinked hyaluronic acid content of 10mg / mL had the highest dynamic viscosity (> 10 0,000mPa.s), which exceeded the upper limit of the measuring instrument measurement and thus was marked as greater than 100,000mPa.s in Figure 1.

The thiol content of the hyaluronic acid thiolated derivative also has a certain effect on the dynamic viscosity of the gel. The gel prepared from the hyaluronic acid thiolated derivative with higher thiol content has a higher dynamic viscosity.

### Example 3: Cytocompatibility Evaluation of Disulfide Crosslinked Hyaluronic Acid Gels

The in vitro cytotoxicity of the disulfide-linked cross-linked hyaluronic acid gels prepared in Example 1 were evaluated with reference to the standards of ISO10993.5-2009.

Rat fibroblasts (ATCC CCL1, NCTC Clone 929, Clone of Strain L) were cultured at 37 ° C, 5% CO₂ and under saturated humidity, using RPMI 1640 medium containing antibiotics (100u / mL penicillin, 100µg / mL streptomycin) and 10% serum. When the cells grow to near confluence, digest with trypsin collect the cells and adjust the cell concentration to 5 × 10⁴ / mL.

RPMI 1640 medium containing 10% serum was used as the extraction medium. 0.2 g of disulfide-linked cross-linked hyaluronic acid gel was added to each ml of the extraction medium. Leaching was performed at 37 ° C for 24 hours. Then the leaching stock solution was diluted by RPMI 1640 with 10% serum to obtain four doses of diluted leaching solutions with the contents of the leaching stock solution of 100%, 50%, 25%, and 12.5%, respectively.

The above cell suspensions was added to a 96-well plate, 100 µL (5 × 10³ cells) per well, and incubated at 37 °C, 5% CO₂ for 24 hours; discard the medium, add the diluted leaching solutions by groups (four doses of 100%, 50%, 25% and 12.5%), negative control, blank control and positive control, 5 holes in each group, incubate in 37 °C, 5% CO₂ saturated steam incubator for 24 hours.

After the incubation, remove the cell culture plate, discard the medium, add 100 µL of RPMI1640 medium containing 10% of serum, add 50 µL (1 mg / mL) of MTT stain to each well, and incubate in 37 °C, 5% CO₂ saturated steam incubator for 3 hours; discard the medium in the culture plate, add 100 µL of isopropanol to each well, mix by shaking, and measure the absorption value at a wavelength of 570 nm.

The relative cell proliferation rate was calculated based on the ratio of the absorption value of each group to the absorption value of the blank control group, and the relative cell proliferation rate of the blank control group was calculated as 100%.

The relative cell proliferation rate of the negative control group was the same as that of the blank control group, and the relative cell proliferation rate of the positive control group was less than 10%, which results were in line with expectations. The relative proliferation rate of cells in each disulfide crosslinked hyaluronic acid gel test group was> 90%, and cytotoxicity was not observed in the cells, indicating that the disulfide crosslinked hyaluronic acid gel tested had good cell compatibility.

### Example 4: Evaluation of In vivo Histocompatibility of Disulfide Crosslinked Hyaluronic Acid Gels

The in vivo histocompatibility of the nine disulfide crosslinked hyaluronic acid gels prepared in Example 1 was evaluated with reference to the standards of ISO10993.6-2007. The disulfide-linked cross-linked hyaluronic acid content of these 9 gels were 3 mg / mL, 6 mg / mL, and 8 mg / mL, respectively, and these nine gels were prepared by hyaluronic acid thiolated derivatives with a thiol content of 24 µmol / g, 38 µmol / g, and 57 µmol / g, respectively. A commercially available non-crosslinked hyaluronic acid gel product was used as a control sample.

After normal disinfection of the skin of healthy SD rats, the disulfide crosslinked hyaluronic acid gels prepared in Example 1 or a control sample (0.5 mL) were implanted subcutaneously along the midline of the spine of the rats (2 cm away from the spine). Rats were sacrificed painlessly 3 days, 7 days, 10 days and 14 days after implantation. The implant and surrounding tissues were cut out for macroscopic observation. The implants and surrounding tissues were placed in 10% formalin for fixation, dehydrated from gradient alcohol, paraffin embedded, sliced, and stained with HE for histopathological observation and evaluation.

Visual observation of the gels and control samples showed slight redness and edema in the wounds of the rats 3 days after implantation, but gradually disappeared as the implantation time increased. Histopathological observation showed that the tissue response of each gel group was not more than mild, similar to the control sample. The results of this test indicate that the disulfide crosslinked hyaluronic acid gels tested have good histocompatibility.

### Example 5: Preparation of Disulfide Crosslinked Hyaluronic Acid Gels

Hyaluronic acid thiolated derivatives are made from sodium hyaluronate with molecular weights of 300 KDa and 1,500 KDa, and are prepared by the method reported by Wang et al (Wang et al, J Mater Chem. B, 2015, 3:7546-7553). Their thiol contents were 103 µmol / g and 75 µmol / g, respectively.

The above-mentioned hyaluronic acid thiolated derivatives were dissolved to obtain aqueous solutions with contents of 3 mg / mL, 4 mg / mL, 4.5 mg / mL, 5 mg / mL, 6 mg / mL, 7 mg / mL, 8 mg / mL and 10 mg / mL. Adjust the pH value of the solutions to 7.4. Transfer the solutions into glass containers and then sterilize the solutions by moist heat. The solutions were stored in a sealed container at room temperature for 4 weeks. The solutions lose fluidity and form disulfide crosslinked hyaluronic acid gels.

### Example 6: Cytocompatibility Evaluation of Disulfide Crosslinked Hyaluronic Acid Gels

The in vitro cytotoxicity of the disulfide-linked cross-linked hyaluronic acid gels prepared in Example 5 were evaluated by the same method as in Example 3.

The relative proliferation rates of cells in each disulfide crosslinked hyaluronic acid gels test group were > 90%, and cytotoxicity was not observed in the cells, indicating that the disulfide crosslinked hyaluronic acid gels tested had good cell compatibility.

### Example 7: Evaluation of In vivo Histocompatibility of Disulfide Crosslinked Hyaluronic Acid Gel

The in vivo tissue compatibility of the four disulfide-linked cross-linked hyaluronic acid gels prepared in Example 5 were evaluated by the same method as in Example 4; two of the gels were prepared by hyaluronic acid thiolated derivatives with a thiol content of 75 µmol / g (1,500KDa), the other two gels were prepared by hyaluronic acid thiolated derivatives with a thiol content of 103 µmol / g (300KDa). The contents of the disulfide crosslinked hyaluronic acid in the gels were 3mg / mL and 4.5mg / mL, respectively. A commercially available non-crosslinked hyaluronic acid gel product was used as a control sample.

Visual observation of the gels and control samples showed slight redness and edema in the wounds of the animals 3 days after implantation, but gradually disappeared as the implantation time increased. Histopathological observation showed that the tissue response of each gel group was not more than mild, similar to the control sample. The results of this test indicate that the disulfide crosslinked hyaluronic acid gels tested had good histocompatibility.

### Example 8: Evaluation of the effect of disulfide crosslinked hyaluronic acid gels on prevention of abdominal (pelvic) cavity adhesions

The classic white rabbit sidewall model (John et al., Fertil Steril. 1997, 68: 37-42) was used to evaluate effects of the disulfide-linked crosslinked hyaluronic acid gels prepared in Example 1 on preventing abdominal (pelvic) cavity adhesions. The disulfide crosslinked hyaluronic acid gels used were prepared from a hyaluronic acid thiolated derivatives with a thiol content of 38 µmol / g, and the contents of the disulfide crosslinked hyaluronic acid in the gels were 3 mg / mL, 4 mg / mL, 4.5 mg / mL, 5 mg / mL, 6 mg / mL, 7 mg / mL, 8 mg / mL, and 10 mg / mL, respectively.

Healthy female white rabbits were anesthetized by intramuscular injections of a mixture of ketamine hydrochloride (55 mg / kg) and lopon (5 mg / kg), and midline laparotomies was performed on the rabbits. Remove the cecum and small intestine, apply pressure to bleed under all serosal surfaces, and then gently rub the damaged intestine with sterile gauze until spotting bleeding is observed. Return the cecum and small intestine to their normal anatomical position. Resect the peritoneums and transverse abdominis muscles with an area of 5 × 3cm² on the right side of the abdominal wall. The resected site was coated with a disulfide crosslinked hyaluronic acid gel (~ 4 mL) or a physiological saline reference. The surgical incision is closed with two layers of absorbable sutures, and be careful when operating to protect the intestine from injury.

The experimental animals were sacrificed 21 days after the operation, and general observations were performed on the open abdomens. At the same time, the percentage of adhesion area and the severity of adhesions involved in side wall injuries were measured and evaluated. The severities of adhesions were scored according to the quartile method: 0 = no adhesions occur; 1 = mild adhesions (adhesion that is easy to peel off); 2 = moderate adhesions (not peelable, do not tear organs); 3 = dense adhesion (not peelable, tearing organs while moving).

Macroscopic observation did not reveal any symptoms of chronic inflammation and granulomatous, indicating that the disulfide crosslinked hyaluronic acid gels tested had good histocompatibility. At the same time, no gel residue was found, indicating that the disulfide crosslinked hyaluronic acid gels tested were completely degraded and absorbed.

The evaluation results are shown in Fig. 2 (percentage of adhesion area) and Fig. 3 (severity of adhesion). The disulfide crosslinked hyaluronic acid gels tested all had adhesion prevention effects, and the gels with contents of 3mg / mL, 4mg / mL, 4.5mg / mL, 5mg / mL, 6mg / mL, 7mg / mL, and 8mg / mL have better adhesion prevention effect (less adhesion area and lower severity of adhesion) than the gel with contents of 10 mg / mL.

### Example 9: Evaluation of the Effect of Disulfide Crosslinked Hyaluronic Acid Gel on Prevention of Abdominal (pelvic) Cavity Adhesions

The classic white rabbit Uterine horn model (John et al., FertilSteril. 1997, 68: 37-42) was used to evaluate the disulfide-linked crosslinked hyaluronic acid gel prepared in Example 1 to prevent abdominal (pelvic) cavity adhesions effect. The disulfide crosslinked hyaluronic acid gels used were prepared from hyaluronic acid thiolated derivatives with a thiol content of 57 µmol / g, and the contents of the disulfide crosslinked hyaluronic acid in the gels were 3 mg / mL, 4 mg / mL, 4.5 mg / mL, 5 mg / mL, 6 mg / mL, 7 mg / mL, 8 mg / mL, and 10 mg / mL, respectively.

Healthy female white rabbits were anesthetized by intramuscular injections of 3% sodium pentobarbital (3mg / kg), and the abdominal cavity was opened along the ventral white line to expose the bilateral uterus and fallopian tubes, and the uterine horn was positioned. The diameter of the uterine horn is measured and recorded. Only white rabbits with a uterine horn diameter of 3 mm or more can continue the test. Using a No. 10 surgical blade, start at the uterine horn, and scrape about 20 times within a range of 1 cm from the fallopian tube and 4 cm from the uterine body until intermittent bleeding on the serous surface can be spotted with naked eyes. Put the scratched uterus and fallopian tubes back to their original natural anatomical sites, and apply 2.5ml of disulfide-linked crosslinked hyaluronic acid gels or normal saline controls on each side of the injuries. Before closing the abdomen, use a syringe to inject 5 mL of disulfide-linked cross-linked hyaluronic acid gel or saline into the abdominal cavity from the tail end of the abdominal wall incision.

The test animals were sacrificed two weeks after the operation, and the abdominal cavities were opened to observe the positions and appearances of the main organs, as well as the general conditions such as peritoneal effusion and residual test samples. Expose the uterus and fallopian tubes, determine the adhesion of the uterine fallopian tubes or the surrounding organs, determine the length of each adhesion, and add the adhesion lengths of the uterine fallopian tubes on both sides as the postoperative adhesion length of the animal.

Rough observation did not reveal any adverse reactions, indicating that the disulfide crosslinked hyaluronic acid gels tested had good histocompatibility. At the same time, no gel residue was found, indicating that the disulfide crosslinked hyaluronic acid gels tested were completely degraded and absorbed.

See Figure 4 for the evaluation results. The disulfide crosslinked hyaluronic acid gels tested all had adhesion prevention effects, and the gels with contents of 3mg / mL, 4mg / mL, 4.5mg / mL, 5mg / mL, 6mg / mL, 7mg / mL, and 8mg / mL had a better adhesion prevention effect (lower adhesion length) than the gel with content of 10 mg / mL.

## Claims

1. A disulfide crosslinked hyaluronic acid gel for preventing tissue adhesion after abdominal operation, **characterized in that** the content of disulfide crosslinked hyaluronic acid is between 3 ~ 4.5 mg / mL.

2. The disulfide crosslinked hyaluronic acid gel for preventing tissue adhesion after abdominal operation according to claim 1, wherein the disulfide crosslinked hyaluronic acid gel is prepared by hyaluronic acid thiolated derivative.

3. The disulfide crosslinked hyaluronic acid gel for preventing tissue adhesion after abdominal operation according to claim 2, wherein thiol content of the hyaluronic acid thiolated derivative is 10 ~ 100 µmol / g.

4. The disulfide crosslinked hyaluronic acid gel for preventing tissue adhesion after abdominal operation according to claim 3, wherein thiol content of the hyaluronic acid thiolated derivative is 20 ~ 70 µmol / g.

5. A method for preparing the disulfide crosslinked hyaluronic acid gel according to any of claims 1-4, wherein the disulfide-crosslinked hyaluronic acid gel is prepared through an oxidation process from a hyaluronic acid thiolated derivative aqueous solution.

6. The method for preparing a disulfide crosslinked hyaluronic acid gel according to claim 5, wherein the oxidation process is performed under the action of oxygen.

7. The method for preparing a disulfide crosslinked hyaluronic acid gel according to claim 6, wherein the oxygen is oxygen in the air and / or oxygen dissolved in aqueous solution.

8. A disulfide crosslinked hyaluronic acid gel according to any one of claims 1-4 for use in a method of preventing tissue adhesion after abdominal operation.

## Patentansprüche

1. Disulfid-vernetztes Hyaluronsäure-Gel zur Verhinderung von Gewebeadhäsion nach einer abdominalen Operation, **dadurch gekennzeichnet, dass** der Gehalt an durch Disulfidvernetzter Hyaluronsäure zwischen 3 - 4,5 mg/mL liegt.

2. Das Disulfid-vernetzte Hyaluronsäure-Gel zur Verhinderung von Gewebeadhäsion nach einer abdominalen Operation gemäß Anspruch 1, wobei das Disulfid-vernetzte Hyaluronsäure-Gel durch ein thioliertes Hyaluronsäurederivat hergestellt wird.

3. Das Disulfid-vernetzte Hyaluronsäure-Gel zur Verhinderung von Gewebeadhäsion nach abdominaler Operation gemäß Anspruch 2, wobei der Thiolgehalt des thiolierten Hyaluronsäurederivats 10 - 100 µmol/g beträgt.

4. Das Disulfid-vernetzte Hyaluronsäure-Gel zur Verhinderung von Gewebeadhäsion nach einer abdominalen Operation gemäß Anspruch 3, wobei der Thiolgehalt des thiolierten Hyaluronsäurederivats 20 - 70 µmol/g beträgt.

5. Verfahren zur Herstellung des Disulfid-vernetzten Hyaluronsäure-Gels gemäß einem der Ansprüche 1-4, wobei das Disulfid-vernetzte Hyaluronsäure-Gel durch ein Oxidationsverfahren aus einer wässrigen Lösung eines thiolierten Hyaluronsäurederivats hergestellt wird.

6. Das Verfahren zur Herstellung eines Disulfid-vernetzten Hyaluronsäure-Gels gemäß Anspruch 5, wobei das Oxidationsverfahren unter Einwirkung von Sauerstoff durchgeführt wird.

7. Das Verfahren zur Herstellung eines Disulfid-vernetzten Hyaluronsäure-Gels gemäß Anspruch 6, wobei der Sauerstoff Sauerstoff in der Luft und/oder in wässriger Lösung gelöster Sauerstoff ist.

8. Disulfid-vernetztes Hyaluronsäure-Gel nach einem der Ansprüche 1-4 zur Verwendung in einem Verfahren zur Verhinderung von Gewebeadhäsion nach einer abdominalen Operation.

## Revendications

1. Gel d'acide hyaluronique réticulé au disulfure pour prévenir l'adhésion tissulaire après une opération abdominale, **caractérisé en ce que** la teneur de l'acide hyaluronique réticulé au disulfure est comprise entre 3 ~ 4,5 mg/ml.

2. Gel d'acide hyaluronique réticulé au disulfure pour prévenir l'adhésion tissulaire après une opération abdominale selon la revendication 1, dans lequel le gel d'acide hyaluronique réticulé au disulfure est préparé par un dérivé thiolé d'acide hyaluronique.

3. Gel d'acide hyaluronique réticulé au disulfure pour prévenir l'adhésion tissulaire après une opération abdominale selon la revendication 2, dans lequel la teneur en thiol du dérivé thiolé d'acide hyaluronique est de 10 ~ 100 pmol/g.

4. Gel d'acide hyaluronique réticulé au disulfure pour prévenir l'adhésion tissulaire après une opération abdominale selon la revendication 3, dans lequel la teneur en thiol du dérivé thiolé d'acide hyaluronique est de 20 ~ 70 pmol/g.

5. Procédé de préparation du gel d'acide hyaluronique réticulé au disulfure selon l'une quelconque des revendications 1 à 4, dans lequel le gel d'acide hyaluronique réticulé au disulfure est préparé à travers un processus d'oxydation à partir d'une solution aqueuse de dérivé thiolé d'acide hyaluronique.

6. Procédé de préparation d'un gel d'acide hyaluronique réticulé au disulfure selon la revendication 5, dans lequel le processus d'oxydation est exécuté sous l'action d'oxygène.

7. Procédé de préparation d'un gel d'acide hyaluronique réticulé au disulfure selon la revendication 6, dans lequel l'oxygène est l'oxygène de l'air et/ou l'oxygène dissous dans une solution aqueuse.

8. Gel d'acide hyaluronique réticulé au disulfure selon l'une quelconque des revendications 1 à 4 pour l'utilisation dans un procédé de prévention de l'adhésion tissulaire après une opération abdominale.
